# EUROPEAN PATENT SPECIFICATION

(11) **EP 1 490 338 B1**
(45) Date of publication and mention of the grant of the patent: **24.08.2011**
(21) Application number: 03714995.2
(22) Date of filing: 03.04.2003
(51) Int. Cl.: C07D 215/00, C07D 239/00, C07D 491/00, A61K 31/435, A61P 25/16

(54) **USE OF AN ALFA2-ADRENORECEPTOR ANTAGONIST FOR CNS-RELATED DISEASES**
VERWENDUNG EINES ALFA2-ADRENOREZEPTORANTAGONISTEN BEI MIT DEM ZNS ZUSAMMENHÄNGENDEN ERKRANKUNGEN
UTILISATION DE L'ANTAGONISTE D'UN ALFA2-ADRENOCEPTEUR EN CAS DE MALADIES DU SNC

(30) Priority: 03.04.2002 US 369323 P
(43) Date of publication of application: 29.12.2004
(73) Proprietor: ORION CORPORATION, 02200 Espoo (FI)
(72) Inventor: SALLINEN, Jukka, FIN-20100 Turku (FI); SIRVIÖ, Jouni, FIN-70820 Kuopio (FI)
(74) Representative: Winter, Brandl, Fürniss, Hübner, Röss, Kaiser, Polte - Partnerschaft
(86) International application number: PCT/FI2003/000254
(87) International publication number: WO 2003/082825

(56) References cited:
- WO-A-00/37466
- WO-A-01/64645
- WO-A-02/18348
- US-A- 4 604 398
- US-A- 4 686 226
- US-A- 6 156 757
- US-A1- 2002 045 614
- US-B1- 6 352 999
- US-B1- 6 426 350
- US-B1- 6 495 555

## Description

### FIELD OF THE INVENTION

The present invention relates to the use of an alfa 2-adrenoceptor antagonist in the manufacture of a pharmaceutical for the treatment of symptoms of disorders and conditions associated with sensorimotor gating deficits. More specifically, a therapeutically effective amount of an alpha2-adrenoceptor antagonist, or its pharmaceutically acceptable ester or salt thereof, selective for the alpha2C-adrenoceptor subtype is administered to a mammal in need of such treatment.

### BACKGROUND OF THE INVENTION

The publications and other materials used herein to illuminate the background of the invention and in particular cases to provide additional details respecting the practice are incorporated by reference.

The startle reflex is a short-latency response of the skeletal musculature elicited by a sudden auditory stimulus. Prepulse-inhibition (PPI) of the startle response refers to the reduction in the startle response caused by a low intensity non-startling stimulus (the prepulse) which is presented shortly before the startle stimulus. PPI can be used as an operational measure of sensorimotor gating and appears to be present in all mammals, including rats and humans (Swerdlow, N. R. et al., The Archives of General Psychiatry 51 (1994) 139-154). Sensorimotor gating i.e. PPI deficits are observed in subgroups of patients with certain neuropsychiatric disorders, such as schizophrenia, obsessive compulsive disorder, Tourette's syndrome, blepharospasm and other focal dystonias, temporal lobe epilepsy with psychosis, drug-induced psychosis (Braff, D. L. et al., Psychopharmacology (Berl) 156(2-3) (2001) 234-258), and panic disorder (Ludewig, M.S. et al, Depression and Anxiety 15 (2002) 55-60). These PPI deficits can be produced in animals by psychostimulants, such as d-amphetamine or phencyclidine (PCP), and reversed by some antipsychotics. The PPI model has been shown to possess high predictive validity and it is therefore widely used in the development of new therapeutic agents (Swerdlow, N. R. et al., The Archives of General Psychiatry 51 (1994) 139-154). Some, but not all, antipsychotics have also weak PPI-enhancing effects *per se.* The mechanisms how PPI is modulated in the CNS are complicated and only partially understood.

The alpha2-adrenoceptors, which include three subtypes (alpha2A, alpha2B, and alpha2C) encoded by three genes, mediate many of the central nervous system (CNS) effects of norepinephrine and regulate the release of several other neurotransmitters in addition to norepinephrine. The alpha2-adrenoceptors are suggested to have modulatory roles in various neuropsychiatric disorders, but their significance in the development of new therapeutics for CNS disorders, especially the role of each alpha2-receptor subtypes is poorly known due to the unavailability of selective ligands for each of the alpha2-adrenoceptor subtypes. However, some hypotheses about the significance of the alpha2-subtypes in CNS disorders has been gained by studies employing mice with genetically altered alpha2-subtype expression, which has produced new hypotheses about the possible actions of alpha2-subtype selective ligands (MacDonald. E. et al., Trends Pharmacol. Sci.18 (1997) 211-219; Scheinin, M. et al., Life Sci 68(19-20) (2001) 2277-85).

Subtype non-selective alpha2-adrenoceptor agonists are known to decrease startle reflex and antagonists to enhance startle reflex. However, the effects of alpha2-agonists or antagonists on the sensorimotor gating phenomenon (i.e. on the prepulse inhibition of startle reflex) are unclear; the general conclusion is that PPI is not altered, but the interpretation is confounded by the effects of alpha2-drugs on startle *per se* (Geyer, M. A. et al., Psychopharmacology (Berl) 156(2-3) (2001) 117-154).

WO-A-2001 64645 discloses the use of alpha-2c adrenoceptor antagonists such as acridin-9-yl-[4-(4-methylpiperazin-1-yl)phenyl]amine for the treatment of Parkinson's disease and schizophrenia.

### SUMMARY OF THE INVENTION

The object of the present invention is to provide a new treatment possibility for symptoms of diseases and conditions characterized by sensorimotor gating deficits. The invention describes how a subtype selective alpha2C-adrenoceptor antagonist, but not subtype non-selective alpha2-adrenoceptor antagonist, enhances sensorimotor gating (i.e. the prepulse-inhibition of startle reflex) *per se* and especially when the normally functioning sensorimotor gating is disrupted by the psychostimulant phencyclidine (PCP).

Additional objects and advantages of the invention will be set forth in part in the description, which follows, and in part will be obvious from the description, or may be learned by practice of the invention. The objects and advantages of the invention will be realized and attained by means of the elements and combinations particularly pointed out in the appended claims.

It is to be understood that the foregoing general description and the following detailed description are exemplary and explanatory only and are not restrictive of the invention as claimed.

### BRIEF DESCRIPTION OF THE DRAWINGS

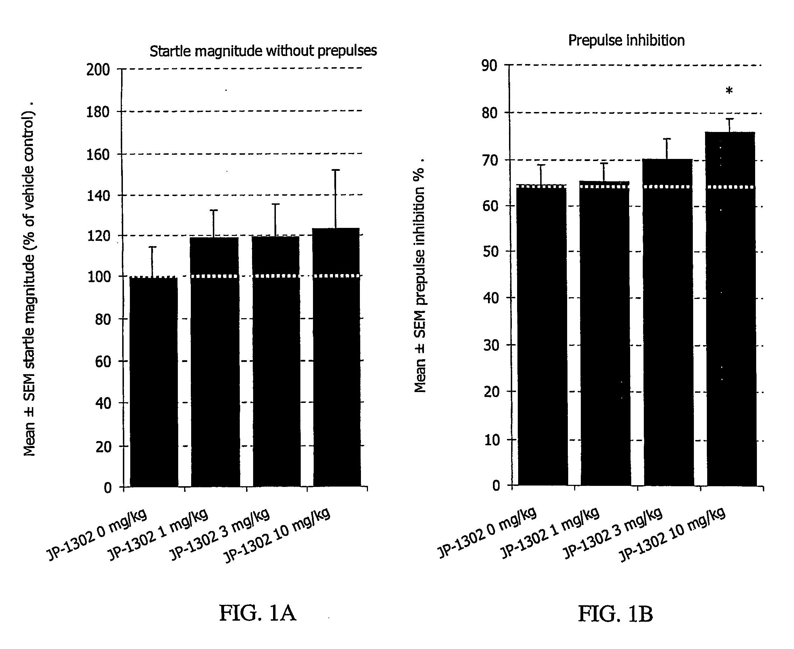

Figures 1A and 1B show the effect of the selective alpha2C-antagonist acridin-9-yl-[4-(4-methylpiperazin-1-yl)-phenyl]amine (JP-1302) (WO 01/64645) on the startle reflex and its prepulse inhibition in rats. The selective alpha2C-antagonist enhanced sensorimotor gating (prepulse inhibition %) without significantly affecting the startle reactivity to intense pulses without prepulses. Asterisk refers to significant difference between vehicle and treatment group p<0.05; 1-way ANOVA and LSD post hoc test.

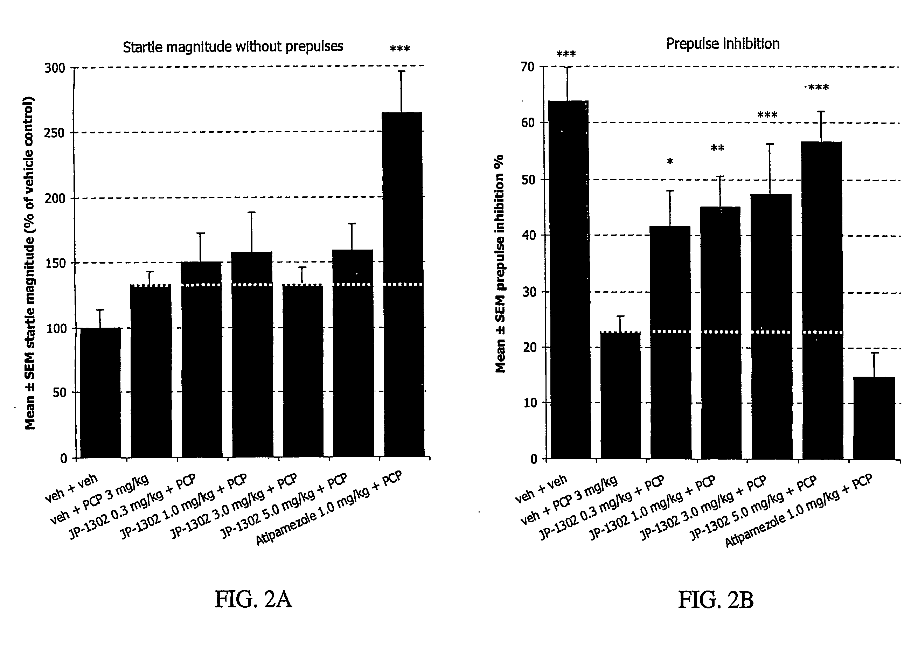

Figures 2A and 2B show the effect of the alpha2C-antagonist acridin-9-yl-[4-(4-methylpiperazin-1-yl)-phenyl]amine (JP-1302) and the alpha2-adrenoceptor subtype non-selective alpha2-antagonist atipamezole on the startle reflex and its prepulse inhibition in rats pretreated with the psychostimulant phencyclidine (PCP). PCP clearly disrupted the PPI and this was effectively counteracted by the subtype selective alpha2C-antagonist, but not by the receptor subtype non-selective alpha2-antagonist atipamezole. Asterisks refer to significant difference in statistical comparisons between the vehicle (veh) + PCP and other treatment groups. **p*< 0.05, ** *p*< 0.01, *** *p*< 0.001; 1-way ANOVA and LSD post hoc test.

### DETAILED DESCRIPTION OF THE INVENTION

The present invention relates to a novel use an alfa2-adrenoceptor antagonist for the manufacture of the pharmaceutical for the treatment of symptoms of disorders and conditions associated with sensorimotor gating deficits in mammals, including humans and animals. The results to be presented below show that a subtype selective alpha2C-adrenoceptor antagonist, but not subtype non-selective alpha2-adrenoceptor antagonist, enhances sensorimotor gating (i.e. the prepulse-inhibition of startle reflex) *per se.*

In a previous study (Sallinen, J. et al., J. Neurosci. 18(1998) 3035-42), alpha2C-knockout mutation was associated with weakened PPI whereas alpha2C-overexpression demonstrated increased PPI. It was therefore speculated, that alpha2C-subtype-selective drugs might have therapeutic value in disorders associated with sensorimotor gating deficits. The novel selective alpha2C-antagonist acridin-9-yl-[4-(4-methylpiperazin-1-yl)-phenyl]amine (JP-1302) has now been tested for its therapeutic value in disorders associated with sensorimotor gating deficits. The results obtained with alpha2C-antagonist turned out to be unexpected; since the previous studies with transgenic mice suggested that an alpha2C-agonist (but not antagonist) would enhance PPI (since overexpression of alpha2C enhanced PPI). Also the magnitude of the effect of the alpha2C-antagonist acridin-9-yl-[4-(4-methylpiperazin-1-yl)-phenyl]amine (JP-1302) can be considered surprising, and the observed effect could not have been anticipated on theoretical basis (genetically altered alpha2C-expression did not affect the PCP -disrupted PPI, Sallinen, J. et al., J. Neurosci. 18(1998) 3035-42).

In order to study the effect of alpha2-antagonists on startle reflex and its prepulse - inhibition, groups of rats (n =10/group) were pre-treated with the alpha2C-antagonist or vehicle 20 min before measurement of the acoustic startle reactivity and PPI in a test system designed for startle studies (SR-LAB, San Diego Instruments, CA, USA). In a subsequent experiment the effects of the alpha2-adrenoceptor subtype selective alpha2C-antagonist and or the subtype non-selective antagonist atipamezole (Haapalinna, A. et al., Naunyn-Schmiedeberg's Arch. Pharmacol. 356(1997) 570-582) on PCP-induced PPI -disruption was studied. The antagonists were given 20 min, and PCP or vehicle 10 min before start of the startle measurements. The method otherwise corresponds to the procedure described in Sallinen, J. et al., J. Neurosci. 18(1998) 3035-42.

It was found that the receptor subtype selective alpha2C-antagonist acridin-9-yl-[4-(4-methylpiperazin-1-yl)-phenyl]amine (JP-1302) did not affect the startle reflex *per se*, but it increased PPI dose-dependently and effectively (Figures 1A and 1B). The effect of the alpha2C-antagonist was especially clearly seen in the presence of PCP (Figures 2A and 2B). In the Figures 2A and 2B it is also shown that the specific and potent alpha2-antagonist atipamezole, that has no alpha2-adrenoceptor subtype selectivity, increased significantly the startle reactivity *per se,* but it had no effect on the PPI phenomenon; this points to the significance of the alpha2C-adrenoceptor subtype selective antagonism.

The present findings suggest that an alpha2C-adrenoceptor selective antagonist can be used to treat symptoms of disorders and conditions associated with sensorimotor gating deficit, particularly symptoms of disorders and conditions wherein the sensorimotor gating deficits results in sensory flooding and cognitive fragmentation causing dysfunction in attention and perception.

Consequently, the present invention relates to the use of an alfa2-adrenoceptor antagonist, or its pharmaceutically acceptable ester or salt thereof, said alfa2-adrenoceptor antagonist being selective for the alfa2C-adrenoceptor subtype, in the manufacture of a pharmaceutical for the treatment of symptoms of disorders or conditions associated with sensorimotor gating deficits, wherein the disorder or condition is obsessive compulsive disorder, Tourette's syndrome, blepharospasm and other focal dystonia, temporal lobe epilepsy with psychosis, drug-induced psychosis, Huntington's disease or panic disorder.

Further, said symptoms, which are usually associated with above-mentioned disorders or conditions include, but are not limited to, hallucination, delusion, parathymia, agitation, psychotic cognitive impairment (including deficits in thinking and speech), social withdrawal and withdrawal symptoms (including delirium) associated with cessation of cigarette smoking or alcohol or drug abuse.

These symptoms may also be seen in animals in exceptional circumstances, for example, during withdrawal from masters or during transportation.

Furthermore, the present invention relates the use of an alfa2-adrenoceptor antagonist, or its pharmaceutically acceptable ester or salt thereof, said alfa2.

adrenoceptor antagonist being selective for the alfa2C-adrenoceptor subtype, in the manufacture of a pharmaceutical for the treatment of symptoms of disorders and conditions associated with sensorimotor gating deficits in a mammal.

For the purposes of the invention the term "treatment" means treatment in order to remedy or alleviate the symptoms of the disorder or condition, and treatment in order to prevent the development or the exacerbation of the symptoms.

For the purposes of the invention the term "alpha2C-selective antagonist" or "an alpha2-adrenoceptor antagonist selective for the alpha2C-adrenoceptor subtype" refers to a compound having no other major affinities for other alpha2-adrenoceptor subtypes than for alpha2C-adrenoceptor subtype. Accordingly, the alpha2-adrenoceptor antagonist should be at least ten-fold more selective for the alpha2C-adrenoceptor subtype than for other alpha2-adrenoceptor subtypes.

Furthermore, the use of an alpha2-adrenoceptor antagonist selective for the alpha2C-adrenoceptor subtype in combination with other psychiatric medication, that is used in conditions in which sensorimotor gating deficits may appear, would be therapeutically beneficial by providing either an effective treatment to patient resistant to the said conventional therapeutic agents alone, or by providing a synergistic action with the said conventional therapeutic agents. Such psychiatric medication include, but is not limited to, an anxiolytic, antidepressive or antipsychotic drug, which drug does not need to have effect on sensorimotor gating deficits.

The alpha2-adrenoceptor antagonist selective for the alpha2C-adrenoceptor subtype and the second compound should preferably be administered to the patient during the same period of treatment. The most preferably, the alpha2-adrenoceptor antagonist selective for the alpha2C-adrenoceptor subtype and the second compound should be administered simultaneously. According to a particularly preferable embodiment, these compounds are administered from the same dosage form.

Such a combination therapy will allow the use of smaller doses of the said compounds and thereby substantially reduce their possible sedative effects, their disturbance on motor functionality, and other adverse effects such as hypotensive effects.

For the purpose of the invention the alpha2-adrenoceptor antagonist; or its pharmaceutically acceptable ester or salt, selective for the alpha2C-adrenoceptor subtype can be administered by various routes. Typical routes of administration include, but are not limited to, oral, transdermal, transmucosal, and parenteral routes. One skilled in the art would recognize the dosage forms suitable in the method of the present invention.

The precise amount of the drug to be administered to a mammal according to the present invention is dependent on numerous factors known to one skilled in the art, such as the compound to be administered, the general condition of the patient, the condition to be treated, the desired duration of the treatment, the type of mammal, the method and route of administration etc. For a subtype selective alfa2C-antagonist the usual daily dosage will be from 1 to 500 mg, preferably from 10 to 30 mg, divided in 1 to 4 individual doses.

## Claims

1. Use of a selective alpha2C-adrenoceptor antagonist, or its pharmaceutically acceptable ester or salt thereof, in the manufacture of a pharmaceutical for the treatment of symptoms of disorders or conditions associated with sensorimotor gating deficits, wherein the disorder or condition is obsessive compulsive disorder, Tourette's syndrome, blepharospasm and other focal dystonia, temporal lobe epilepsy with psychosis, drug-induced psychosis, Huntington's disease or panic disorder.

2. Use according to claim 1 wherein the disorder is obsessive compulsive disorder.

3. Use according to claim 1, wherein the disorder is Tourette's syndrome.

4. Use according to any one of Claims 1 to 3, wherein the symptom is hallucination, delusion, parathymia, agitation, psychotic cognitive impairment, social withdrawal and/or withdrawal symptom associated with cessation of cigarette smoking or alcohol or drug abuse.

5. Use according to claim 4, wherein the symptom is hallucination.

6. Use according to claim 4, wherein the symptom is delusion.

7. Use according to claim 4, wherein the symptom is parathymia.

8. Use according to claim 4, wherein the symptom is agitation.

9. Use according to claim 4, wherein the symptom is psychotic cognitive impairment.

10. Use according to claim 4, wherein the symptom is social withdrawal.

11. Use according to any one of claims 1 to 10, wherein the pharmaceutical is for treatment in combination with other psychiatric medication.

12. Use according to any one of claims 1 to 11, wherein the pharmaceutical is for treatment in a human.

## Patentansprüche

1. Verwendung eines selektiven alpha2c-Adrenoceptor-Antagonisten, oder seines pharmazeutisch verträglichen Esters oder des Salzes davon in der Herstellung eines Medikaments zur Behandlung von Symptomen oder Störungen oder Zuständen, die mit Reizverarbeitungsdefiziten assoziiert sind, wobei die Störung oder der Zustand eine Zwangsneurose, Tourettesyndrom, Blepharospasmus oder eine weitere fokale Dystonie, Temporallappenepilepsie mit Psychose, eine medikamenteninduzierte Psychose, Chorea Huntington oder eine Panikstörung ist.

2. Verwendung nach Anspruch 1, wobei die Störung eine Zwangsneurose ist.

3. Verwendung, nach Anspruch 1, wobei die Störung Tourettesyndrom ist.

4. Verwendung nach einem der Ansprüche 1 bis 3, wobei das Symptom Halluzination, Wahnvorstellung, Parathymie, Unruhe, psychotische kognitive Beeinträchtigung, sozialer Rückzug und/oder eine Entzugserscheinung, die mit dem Aufhören von Zigarettenrauchen oder Alkohol- oder Drogenmissbrauch assoziiert ist, ist.

5. Verwendung nach Anspruch 4, wobei das Symptom Halluzination ist.

6. Verwendung nach Anspruch 4, wobei das Symptom Wahnvorstellung ist.

7. Verwendung nach Anspruch 4, wobei das Symptom Parathymie ist.

8. Verwendung nach Anspruch 4, wobei das Symptom Unruhe ist.

9. Verwendung nach Anspruch 4, wobei das Symptom psychotische kognitive Beeinträchtigung ist.

10. Verwendung nach Anspruch 4, wobei das Symptom sozialer Rückzug ist.

11. Verwendung nach einem der Ansprüche 1 bis 10, wobei das Medikament zur Behandlung in Kombination mit weiterer psychiatrischer Medikation bestimmt ist.

12. Verwendung nach einem der Ansprüche 1 bis 11, wobei das Medikament zur Behandlung in einem Menschen bestimmt ist.

## Revendications

1. Utilisation d'un antagoniste sélectif des récepteurs adrénergiques alpha-2c, ou de son ester ou sel pharmaceutiquement acceptable, dans la fabrication d'un produit pharmaceutique destiné au traitement de symptômes de troubles ou d'affections associés à des déficiences de filtrage sensoriel, où le trouble ou l'affection est un trouble obsessionnel compulsif, le syndrome de Tourette, un blépharospasme et une autre dystonie focale, une épilepsie du lobe temporal avec psychose, une psychose induite par un médicament, la maladie de Huntington ou un trouble de panique.

2. Utilisation selon la revendication 1, où le trouble est un trouble obsessionnel compulsif.

3. Utilisation selon la revendication 1, où le trouble est le syndrome de Tourette.

4. Utilisation selon l'une quelconque des revendications 1 à 3, où le symptôme est une hallucination, une illusion, une parathymie, une agitation, une altération psychotique de la cognition, un retrait social et/ou un symptôme de sevrage associé à un arrêt du tabac ou d'un abus d'alcool ou d'une toxicomanie.

5. Utilisation selon la revendication 4, où le symptôme est une hallucination.

6. Utilisation selon la revendication 4, où le symptôme est une illusion.

7. Utilisation selon la revendication 4, où le symptôme est une parathymie.

8. Utilisation selon la revendication 4, où le symptôme est une agitation.

9. Utilisation selon la revendication 4, où le symptôme est une altération psychotique de la cognition.

10. Utilisation selon la revendication 4, où le symptôme est un retrait social.

11. Utilisation selon l'une quelconque des revendications 1 à 10, où le produit pharmaceutique est destiné à un traitement en combinaison avec un autre médicament psychiatrique.

12. Utilisation selon l'une quelconque des revendications 1 à 11, où le produit pharmaceutique est destiné à un traitement chez un être humain.
